# EUROPEAN PATENT SPECIFICATION

(11) **EP 4 135 820 B1**
(45) Date of publication and mention of the grant of the patent: **25.03.2026**
(21) Application number: 21788881.7
(22) Date of filing: 07.04.2021
(51) Int. Cl.: A61M 25/02, A61M 25/06, A61M 25/00, A61M 39/02

(54) **CATHETER ASSEMBLY**
KATHETERANORDNUNG
ENSEMBLE CATHÉTER

(30) Priority: 14.04.2020 SE 2050419
(43) Date of publication of application: 22.02.2023
(73) Proprietor: Vigmed AB, 252 21 Helsingborg (SE)
(72) Inventor: HELLSTRÖM, Maria Cecilia Therese, 234 42 Lomma (SE); HANNER, Gert, 263 94 Mjöhult (SE); THÖRNE, Johan Fredrik, 254 39 Helsingborg (SE)
(74) Representative: Burger, Hannes Alfred
(86) International application number: PCT/SE2021/050310
(87) International publication number: WO 2021/211035

(56) References cited:
- US-A- 5 370 624
- US-A- 5 370 624
- US-A1- 2002 177 814
- US-A1- 2011 224 617
- US-A1- 2014 221 932
- US-A1- 2014 221 932
- US-A1- 2017 120 009
- US-A1- 2019 275 297
- US-A1- 2019 275 297
- US-A1- 2019 307 989

## Description

### TECHNICAL FIELD

The present invention relates to a catheter assembly having a catheter hub rotatable part. Further, the invention relates to a catheter instrument, comprising said catheter assembly and a needle assembly.

### BACKGROUND

Intravenous catheters are used to administer fluids and/or drugs to patients regularly. Fluids are supplied through the catheter through an extension tube, coupled to the catheter through an extension tube coupling present and extending from the catheter hub body.

When a catheter instrument is to be applied to a patient, by for instance a caretaker, a needle extending through the catheter tube is used to insert the catheter intravenously. Once the catheter tube is in place, the needle is extracted and the catheter tube remains intravenously.

However, a common issue when applying catheters to various positions on the patient, is that features of the catheter instrument obstruct the application process for the caretaker. Alternatively, the patient is surrounded by additional medical equipment, forcing the application of the catheter to be conducted using only either the right or left hand. Moreover, intravenous catheters are in general adapted for use by right handed people.

In US 20170120009 A1, the grip of the needle assembly is moveable in relation to the extension tube of the catheter, in an attempt to make the catheter instrument more adaptable to different situations. However, this solution still does not mitigate the above mentioned issues. US 20190275297 A1 discloses a catheter assembly with a rotatable flow housing, but the structure of the catheter comprises various parts. Hence, the production process is complex and cumbersome. US 2014/221932 A1 and US 2019/275297 A1 disclose further catheter assemblies with rotatable parts.

Hence, an improved catheter assembly which is easier to use and simple to manufacture is desired.

### SUMMARY

It is an object of the present invention, considering the disadvantages mentioned above, to provide a catheter assembly according to claim 1.

In a second aspect, there is provided a catheter instrument, comprising the catheter assembly and a needle assembly.

Further features of the invention and its embodiments are set forth in the appended claims.

### BRIEF DESCRIPTION OF THE DRAWINGS

These and other aspects, features and advantages of which the invention is capable will be apparent and elucidated from the following description of non-limiting embodiments of the present invention, reference being made to the accompanying drawings, in which
Fig. 1a is an isometric view of a catheter assembly having an extension tube in a left hand side configuration;
Fig. 1b is the catheter assembly shown in Fig. 1a seen from above;
Fig. 2a is an isometric view of the catheter assembly having the extension tube in an intermediate configuration;
Fig. 2b is the catheter assembly shown in Fig. 2a seen from above;
Fig. 3a is an isometric view of a catheter assembly having an extension tube in a right hand side configuration;
Fig. 3b is the catheter assembly shown in Fig. 3a seen from above; and
Fig. 4 is a longitudinal cross section of the catheter assembly.

### DETAILED DESCRIPTION OF THE EMBODIMENTS

Embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

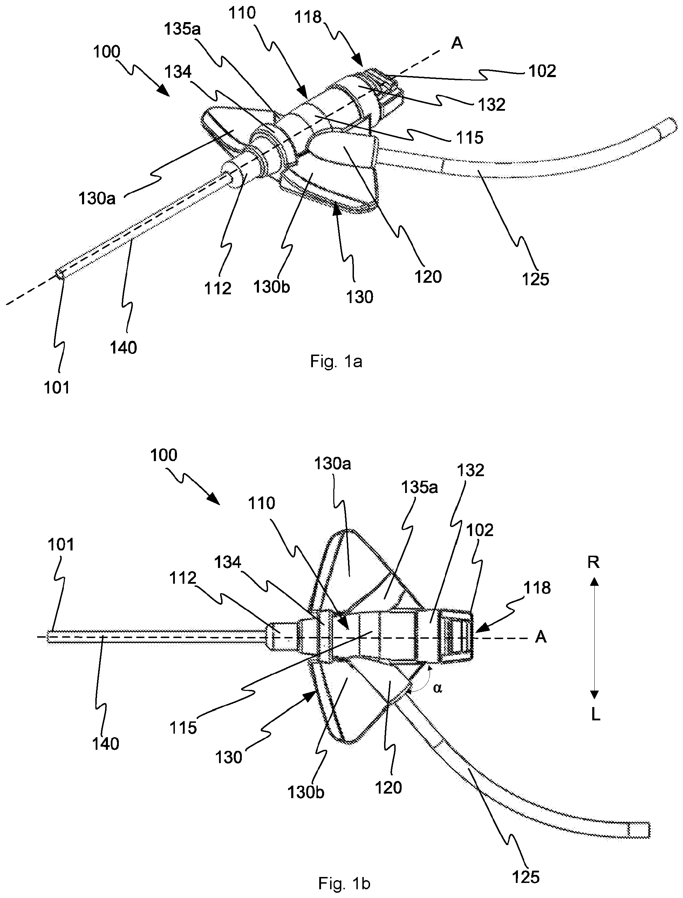

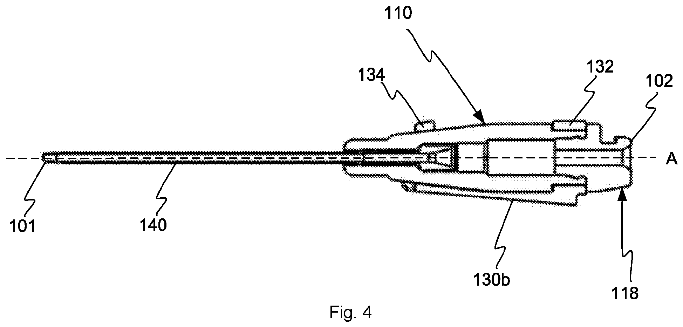

Embodiments of the present invention will now be described below with reference to Figs. 1 to 4.

Referring to Figures 1a to 3b, there is provided a catheter assembly 100 comprising a catheter hub 110, an extension tube coupling 120, also referred to as a coupling element 120 herein, a fixation element in the form of a pair of wings 130 and a hollow catheter tube 140. The hollow catheter tube 140 is configured to house a needle (not shown).

The catheter assembly 100 extends along a longitudinal central axis A in a proximodistal direction. The catheter assembly 100 has a catheter proximal end 101, facing towards the catheter tube 140 and an oppositely facing catheter distal end 102.

A right hand side R is defined as the right lateral side of the axis A when viewing the catheter assembly from the distal end 102. A left hand side L is defined as the left lateral side of the axis A when viewing the catheter assembly 100 from the proximal end 102. The right versus left lateral directions are indicated in Figs. 1b, 2b and 3b with a double ended arrow.

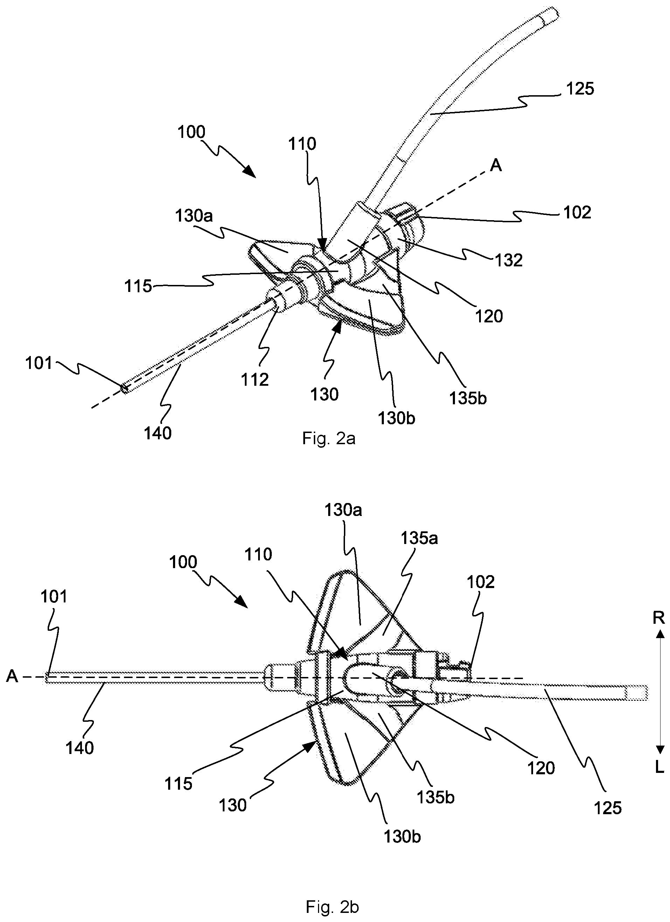

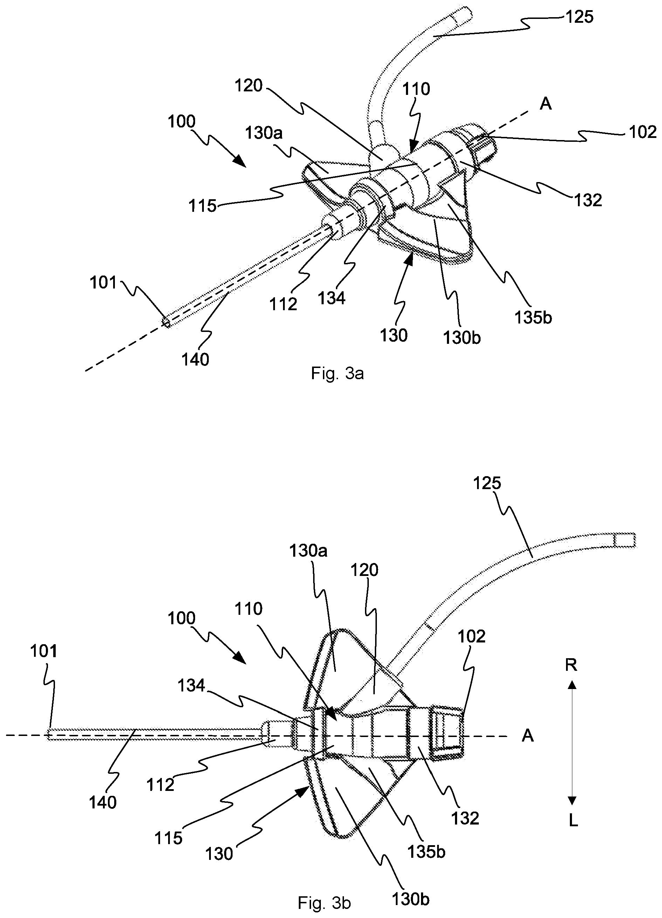

The catheter assembly 100 disclosed herein has a left hand side configuration (shown in Figs. 1a and 1b), an intermediate configuration (shown in Figs. 2a and 2b) and a right hand side configuration (shown in Figs. 3a and 3b).

In turn, the catheter hub 110 comprises a catheter hub base part 112 and a catheter hub rotable part 115. The catheter hub base part 112 is also referred to as a catheter hub front part 112 herein. At the catheter distal end 102, the catheter hub 110 is equipped with a connection portion 118, to which a needle shield assembly (not shown) may be coupled.

The extension tube coupling 120 is connected to the catheter hub rotable part 115, and an extension tube 125 is attached to the coupling element 120. Further, the coupling means 120 may be connected to other features than the extension tube 125.

Preferably, the extension tube coupling 120 extends from the catheter hub 110 at an angle α between the extension tube coupling 120 and the distal end 102 of the catheter hub 110. The angle α shown in Fig. 1b is smaller than 90 degrees. However, the angle α may be 160 degrees or smaller than 160 degrees, such as for instance 140, 120, 100, 80, 70, 60, 45 or 30 degrees.

The catheter hub rotable part 115 is rotatable about said axis A and in relation to the catheter hub front part 112. Hence, when the catheter hub rotable part 115 is moved and turned about the axis A, the extension tube coupling 120 and the extension tube 125 attached thereto will also be displaced about the axis A in relation to the catheter hub front part 112 and in relation to the pair of wings 130. The catheter tube 140 is connected to the catheter hub front part 112 and will thus not rotate together with the catheter hub rotable part 115.

The pair of wings 130 extends laterally to the right hand side R and to the left hand side L from the central axis A. The pair of wings 130 is formed from a right wing 130a and a right wing 130b.

The wing pair 130 comprises a distal coupling means 132 and a proximal coupling means 134. Both coupling means 132, 134 are in the form of an aperture formed integrally with the pair of wings 130, and which each circumvents the catheter hub 110 and joins the catheter hub 110 and the pair of wings 130 together.

### Left hand side configuration

With reference to Figs. 1a and 1b, the left hand side configuration of the catheter assembly 100 will be further explained. The extension tube coupling 120 is arranged on a left hand side L of the axis A.

As shown in Fig. 1b, the right wing 130a of the pair of wings 130 is provided with a right wing recess 135a, also referred to as a right recess 135a herein. The right wing recess 135a is also partly visible in Fig. 1a (but is partly covered by the catheter hub 110 due to the perspective of Fig. 1a). The left wing 130b of the wing pair 130 is also provided with such recess, being a left wing recess 135b (shown in Figs. 2a to 3b), also referred to as a left recess 135b herein. However, the extensions tube coupling 120 is fitted in said left wing recess 135b, and hence cannot be seen in Figs 1a and 1b. The left and right wing recesses 135a, 135b are shaped and designed such that the extension tube coupling 120 will fit within the recesses 135a, 135b.

### Intermediate configuration

The catheter assembly 100 is shown in its intermediate configuration in Figs 2a and 2b. The catheter hub rotable part 115 has been moved approximately 90 degrees about the axis A from the left hand side L towards the right hand side R. By turning the catheter hub rotable part 115 additionally approximately 90 degrees about the axis A towards the right hand side R from the intermediate configuration, the catheter assembly 100 will conform into its right hand side configuration. The intermediate configuration is a type of transformation state between the left hand side and right hand side configurations, which are the two optional configurations available for the user. Both the right wing recess 135a and the left wing recess 135b are visible in Fig. 2b.

### Right hand side configuration

The right hand side configuration is shown in Figs 3a and 3b. The catheter hub rotable part 115 has been moved about the axis A approximately 180 degrees from its left hand side configuration to its right hand side configuration. The extension tube coupling 120 is fitted in the right hand side recess 135a (shown in Figs 1a-2b).

The catheter assembly 100 may further comprise a seal (not shown) between the catheter hub rotable part 115 and the catheter hub front part 112 to avoid liquids to penetrate and/or leak out of the catheter hub 110. Such seal may be in the form of a round elastomeric seal.

### Second embodiment - catheter tube movable with rotable part

In a second embodiment, the catheter hub rotable part 115 and the catheter hub front part 112 are integral with one another, such that the catheter hub front part 112 rotates together with the catheter hub rotable part 115. In such case, the catheter tube 140 will also rotate when the rotatable part 115 rotates about the axis A.

### Common features

The rotation of the catheter rotable part 115 can be conducted either before or after the catheter assembly 100 has been connected to a patient. This facilitates the use of the catheter assembly 100 for left and right handed users and/or caretakers.

The disclosed catheter assembly 100 is a closed system, i.e. a system wherein a septum is provided in the catheter hub 111, and fluids are withdrawn or introduced through the lateral extension tube 125. In such systems, the extension tube coupling 120 is commonly placed on a left hand side L, since this simplifies the use of the catheter assembly 100 for right handed people, which in general represent the majority of people. However, for left handed people, the use of such catheter devices are cumbersome and inconvenient.

The pair of wings 130 disclosed herein will act as a fixation element when the catheter assembly 100 is to be attached to the patient. The catheter assembly 100 is for instance attached by covering the wing pair 130 with medical tape which is attached to the skin of the patient. However, such fixation element may have another shape than that of the wing pair 130 and be used together with the catheter hub 110 having a catheter rotable part 115 as disclosed herein. For instance, each wing of the pair of wings 130 may have a round or rectangular shape.

The rotation angle of the catheter rotable part 115 about the axis A depend on the flexibility of the pair of wings 130 and the surface which they are attached to. For instance, when using a pair of wings 130 of a highly flexible material and when the wings 130 are attached to a thin object, such as a child's finger, the rotation angle may be as high as 320 degrees.

The pair of wings 130 are made of a flexible, resilient material, for instance a plastic (such as an elastomer) or a silicone.

Moreover, the distal coupling means 132 and the proximal coupling means 134 need not be integrally formed with the wing pair 130, but may be formed separately from the wing pair 130 and connected to the pair of wings 130 and the catheter hub 110 respectively. Also, in one embodiment, the wing pair 130 only comprises one coupling means 132, 134.

Further, the catheter assembly 100 may be connected through the connection portion 118 to a needle assembly (not shown) to form a catheter instrument. Such needle assembly may be any needle assembly known in the art.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. **In** addition, singular references do not exclude a plurality. The terms "a", "an", "first", "second" etc. do not preclude a plurality. Reference signs in the claims are provided merely as a clarifying example and shall not be construed as limiting the scope of the claims in any way.

## Claims

1. A catheter assembly, extending along a longitudinal axis A and comprising
a catheter hub (110), comprising a base part (112), a catheter hub rotable part (115) and a coupling element (120) connected to the catheter hub rotatable part (115); and
a fixation element (130) connected to the catheter hub (110), wherein the fixation element (130) in the form of a pair of wings comprises a right wing (130a) and a left wing (130b), wherein the right wing (130a) comprises a right wing recess (135a) configured to fit the coupling element (120), and the left wing (130b) comprises a left wing recess (135b) configured to fit the coupling element (120), such that the coupling element (120) fits within the right wing recess (135a) or the left wing recess (135b),
wherein
the fixation element (130) further comprises at least one coupling means (132, 134) formed integrally with said fixation element (130) and coupling the fixation element (130) to the catheter hub (110),
**characterized in that** the catheter hub rotatable part (115) is rotatable about the axis A in relation to the fixation element (130) and in relation to the base part (112).

2. The catheter assembly according to claim 1, wherein an extension tube (125) is connected to the coupling element (120).

3. The catheter assembly according to claim 2, wherein said coupling means (132, 134) is an opening circumventing the catheter hub (110).

4. The catheter assembly according to any one of the preceding claims,
wherein an angle (α) between the coupling means (120) and a distal end (102) of the catheter hub (110) is 160 degrees or smaller than 160 degrees.

5. The catheter assembly according to any one of the preceding claims,
wherein the catheter hub (110) further comprises a connection part (118) at a distal end (102) thereof, configured to be connected to a needle assembly before said catheter assembly (100) is coupled to a patient.

6. The catheter assembly according to any one of the preceding claims,
wherein the fixation element (130) is made of a flexible, resilient material, preferably silicone or a plastic.

7. The catheter assembly according to any one of the preceding claims,
wherein the catheter hub rotable part (115) is rotatable about the axis A between 0 to 320 degrees, preferably between 0 and 200 degrees, most preferred between 0 and 180 degrees.

8. The catheter assembly according to any one of the preceding claims,
wherein a catheter tube (140) is attached to the catheter hub base part (112) formed integrally with the catheter hub rotable part (115).

9. The catheter assembly according to any one of the claims 1 to 7,
wherein a catheter tube (140) is attached to the catheter hub base part (112), the catheter hub base part (112) being separate from the catheter hub rotable part (115), whereby the rotable part (115) is rotatable relative the fixation element (130) and the catheter hub base part (112) is fixed relative the fixation element (130).

10. The catheter assembly according to claim 9, wherein the catheter hub (110) further comprises a seal between the catheter hub rotable part (115) and the catheter hub base part (112).

11. A catheter instrument, comprising the catheter assembly (100) according to any one of the claims 1 to 10, and a needle assembly.

## Patentansprüche

1. Katheteranordnung, die sich entlang einer Längsachse A erstreckt und Folgendes umfasst:
eine Katheternabe (110), die ein Basisteil (112), ein drehbares Katheternabenteil (115) und ein mit dem drehbaren Katheternabenteil (115) verbundenes Kupplungselement (120) umfasst; und
ein Befestigungselement (130), das mit der Katheternabe (110) verbunden ist,
wobei das Befestigungselement (130) in Form eines Flügelpaares einen rechten Flügel (130a) und einen linken Flügel (130b) umfasst,
wobei der rechte Flügel (130a) eine rechte Flügelaussparung (135a) aufweist, die so gestaltet ist, dass sie zu dem Kupplungselement (120) passt, und der linke Flügel (130b) eine linke Flügelaussparung (135b) aufweist, die so gestaltet ist, dass sie zu dem Kupplungselement (120) passt, so dass das Kupplungselement (120) in die rechte Flügelaussparung (135a) oder in die linke Flügelaussparung (135b) passt,
wobei das Befestigungselement (130) ferner zumindest ein Kupplungsmittel (132, 134) umfasst, das integral mit dem Befestigungselement (130) ausgebildet ist und das Befestigungselement (130) mit der Katheternabe (110) koppelt,
**dadurch gekennzeichnet, dass** das drehbare Katheternabenteil (115) um die Achse A in Bezug auf das Befestigungselement (130) und in Bezug auf das Basisteil (112) drehbar ist.

2. Katheteranordnung nach Anspruch 1, wobei ein Verlängerungsschlauch (125) mit dem Kupplungselement (120) verbunden ist.

3. Katheteranordnung nach Anspruch 2, wobei das Kupplungsmittel (132, 134) eine Öffnung ist, die die Katheternabe (110) umgibt.

4. Katheteranordnung nach einem der vorhergehenden Ansprüche,
wobei ein Winkel (α) zwischen dem Kupplungsmittel (120) und einem distalen Ende (102) der Katheternabe (110) 160 Grad oder weniger als 160 Grad beträgt.

5. Katheteranordnung nach einem der vorhergehenden Ansprüche,
wobei die Katheternabe (110) ferner ein Verbindungsteil (118) an ihrem distalen Ende (102) umfasst, das dazu ausgelegt ist, mit einer Nadelanordnung verbunden zu werden, bevor die Katheteranordnung (100) mit einem Patienten verbunden wird.

6. Katheteranordnung nach einem der vorhergehenden Ansprüche,
wobei das Befestigungselement (130) aus einem flexiblen, elastischen Material, vorzugsweise Silikon oder einem Kunststoff, besteht.

7. Katheteranordnung nach einem der vorhergehenden Ansprüche,
wobei das drehbare Katheternabenteil (115) um die Achse A zwischen 0 und 320 Grad, vorzugsweise zwischen 0 und 200 Grad, besonders bevorzugt zwischen 0 und 180 Grad, drehbar ist.

8. Katheteranordnung nach einem der vorhergehenden Ansprüche,
wobei ein Katheterschlauch (140) am Basisteil (112) der Katheternabe angebracht ist, welches Basisteil (112) integral mit dem drehbaren Katheternabenteil (115) ausgebildet ist.

9. Katheteranordnung nach einem der Ansprüche 1 bis 7,
wobei ein Katheterschlauch (140) am Basisteil (112) der Katheternabe angebracht ist, wobei das Basisteil (112) der Katheternabe vom drehbaren Katheternabenteil (115) getrennt ist, wodurch das drehbare Teil (115) relativ zum Befestigungselement (130) drehbar ist und das Basisteil (112) der Katheternabe relativ zum Befestigungselement (130) fixiert ist.

10. Katheteranordnung nach Anspruch 9, wobei die Katheternabe (110) ferner eine Dichtung zwischen dem drehbaren Katheternabenteil (115) und dem Basisteil (112) der Katheternabe aufweist.

11. Katheterinstrument, umfassend die Katheteranordnung (100) nach einem der Ansprüche 1 bis 10 und eine Nadelanordnung.

## Revendications

1. Ensemble cathéter, s'étendant le long d'un axe longitudinal A et comprenant :
un moyeu de cathéter (110), comprenant une partie de base (112), une partie rotative de moyeu de cathéter (115) et un élément de couplage (120) relié à la partie rotative de moyeu de cathéter (115) ; et
un élément de fixation (130), relié au moyeu de cathéter (110),
l'élément de fixation (130) sous la forme d'une paire d'ailes comprend une aile droite (130a) et une aile gauche (130b),
dans lequel l'aile droite (130a) comprend un évidement d'aile droite (135a) configuré pour s'ajuster à l'élément de couplage (120), et l'aile gauche (130b) comprend un évidement d'aile gauche (135b) configuré pour s'ajuster à l'élément de couplage (120), de telle sorte que l'élément de couplage (120) s'ajuste à l'intérieur de l'évidement d'aile droite (135a) ou de l'évidement d'aile gauche (135b),
dans lequel l'élément de fixation (130) comprend en outre au moins des moyens de couplage (132, 134) formés d'un seul tenant avec ledit élément de fixation (130) et couplant l'élément de fixation (130) au moyeu de cathéter (110),
**caractérisé en ce que** la partie rotative de moyeu de cathéter (115) peut tourner autour de l'axe A par rapport à l'élément de fixation (130) et par rapport à la partie de base (112).

2. Ensemble cathéter selon la revendication 1, dans lequel un tube d'extension (125) est relié à l'élément de couplage (120).

3. Ensemble cathéter selon la revendication 2, dans lequel lesdits moyen de couplage (132, 134) sont une ouverture faisant le tour du moyeu de cathéter (110).

4. Ensemble cathéter selon l'une quelconque des revendications précédentes, dans lequel un angle (α) entre les moyens de couplage (120) et une extrémité distale (102) du moyeu de cathéter (110) est de 160 degrés ou inférieur à 160 degrés.

5. Ensemble cathéter selon l'une quelconque des revendications précédentes, dans lequel le moyeu de cathéter (110) comprend en outre une partie de liaison (118) à une extrémité distale (102) de celui-ci, configurée pour être reliée à un ensemble d'aiguille avant que ledit ensemble cathéter (100) ne soit couplé à un patient.

6. Ensemble cathéter selon l'une quelconque des revendications précédentes, dans lequel l'élément de fixation (130) est constitué d'un matériau flexible et élastique, de préférence du silicone ou un plastique.

7. Ensemble cathéter selon l'une quelconque des revendications précédentes, dans lequel la partie rotative de moyeu de cathéter (115) peut tourner autour de l'axe A sur 0 à 320 degrés, de préférence sur 0 à 200 degrés, de la manière la plus préférée sur 0 à 180 degrés.

8. Ensemble cathéter selon l'une quelconque des revendications précédentes, dans lequel un tube de cathéter (140) est fixé à la partie de base de moyeu de cathéter (112) formé d'un seul tenant avec la partie rotative de moyeu de cathéter (115).

9. Ensemble cathéter selon l'une quelconque des revendications 1 à 7, dans lequel un tube de cathéter (140) est fixé à la partie de base de moyeu de cathéter (112), la partie de base de moyeu de cathéter (112) étant séparée de la partie rotative de moyeu de cathéter (115), moyennant quoi la partie rotative (115) est rotative par rapport à l'élément de fixation (130) et la partie de base de moyeu de cathéter (112) est fixe par rapport à l'élément de fixation (130).

10. Ensemble cathéter selon la revendication 9, dans lequel le moyeu de cathéter (110) comprend en outre un joint d'étanchéité entre la partie rotative de moyeu de cathéter (115) et la partie de base de moyeu de cathéter (112).

11. Instrument à cathéter, comprenant l'ensemble cathéter (100) selon l'une quelconque des revendications 1 à 10, et un ensemble d'aiguille.
